# FASCICULE DE BREVET EUROPEEN

(11) **EP 2 019 706 B1**
(45) Date de publication et mention de la délivrance du brevet: **21.10.2015**
(21) Numéro de dépôt: 07766082.7
(22) Date de dépôt: 22.05.2007
(51) Int. Cl.: A61M 39/10

(54) **CONNECTEUR LUER LOCK MALE**
LUER-LOCK-STECKER
MALE LUER LOCK CONNECTOR

(30) Priorité: 24.05.2006 FR 0604659
(43) Date de publication de la demande: 04.02.2009
(73) Titulaire: CAIR L.G.L., 69380 Lissieu (FR)
(72) Inventeur: LOPEZ, Georges-Antoine, F-69290 Craponne (FR); DELORME, Patrick, F-69630 Chaponost (FR); ALLARD, Ludovic, F-69390 Millery (FR)
(74) Mandataire: Cabinet Laurent & Charras
(86) Numéro de dépôt international: PCT/FR2007/051308
(87) Numéro de publication internationale: WO 2007/135337

(56) Documents cités:
- DE-A1- 3 737 665
- DE-U1- 8 321 694
- FR-A- 2 861 311
- US-A- 4 875 718
- US-A- 5 549 583
- US-A- 6 059 325
- US-A1- 2006 005 840
- US-A1- 2006 033 334

## Description

L'invention a pour objet un connecteur à usage médical. Elle concerne plus particulièrement un connecteur luer lock mâle destiné à coopérer avec un connecteur luer femelle de sorte à raccorder un conduit de liquide.

On trouve aujourd'hui sur le marché, deux types de connecteur luer lock.

La première gamme est constituée par des connecteurs dans lesquels un raccord luer lock mâle est monté en rotation sur un corps tubulaire solidaire de la tubulure d'arrivée de fluide. Dans ce type de connecteur, le raccord n'est pas mobile en translation, seul le mouvement de rotation étant possible. En pratique, le raccord est pourvu d'un filetage permettant le vissage sur l'embout luer femelle. Pour garantir l'étanchéité, on prévoit un joint torique positionné entre la surface interne du raccord et la surface externe du corps. Cette forme de connecteur est par exemple décrite dans le document DE 373 76 65.

L'autre gamme de connecteur est par exemple celle illustrée dans le document EP-A-1 236 481. A la différence des connecteurs luer lock décrits ci-avant, le raccord est ici non seulement mobile en rotation sur le corps tubulaire mais également mobile en translation le long du corps. Une butée est ménagée sur le corps de sorte à limiter la course de la bague et par conséquent sa désolidarisation avec le corps. Le serrage avec le luer femelle se fait aussi par vissage. Ce type de connecteur présente un inconvénient majeur. En effet, la bague mobile, lorsqu'elle est serrée, c'est-à-dire lorsqu'il y a jonction entre le luer mâle et le luer femelle, a tendance à se desserrer du fait de la contrainte entre les filets internes de la bague et la butée limitant son mouvement de translation. En d'autres termes, la bague a tendance à se dévisser et ne joue alors plus sa fonction de verrouillage. Il s'ensuit un risque de fuites, voire même de déconnexion.

L'invention a pour objet un perfectionnement de la première gamme de connecteurs. Les dispositifs décrits dans le document DE 373 76 65 présentent un certain nombre d'inconvénients. Tout d'abord, le risque de déconnexion du luer mâle par rapport au corps subsiste. En effet, la solidarisation du raccord luer mâle avec le corps est obtenue dans ce document par clipsage du connecteur luer mâle au moyen d'un bourrelet ménagé sur toute la périphérie du corps. En fonction de la pression appliquée dans le connecteur, il peut se produire une déconnexion du système du fait de la forme arrondie dudit bourrelet. Par ailleurs, le coût de ce type de produit est élevé en raison de la présence de trois pièces respectivement un corps, un raccord et un joint. Le document US 5 549 583 décrit également un connecteur comportant un corps, un raccord et un joint.

En d'autres termes, le problème que se propose de résoudre l'invention est de mettre au point un connecteur luer lock qui soit dépourvu de joint de manière à diminuer le coût du produit, mais qui reste malgré tout parfaitement étanche et dépourvu de tout risque de déconnexion de la bague par rapport au corps.

Pour résoudre ce problème, le Demandeur a mis au point une forme spécifique du corps, coopérant par friction avec une forme correspondante ménagée à l'extrémité du raccord et ce en l'absence d'un quelconque joint.

Plus précisément, l'invention a pour objet un connecteur à usage médical comprenant :
- un corps solidaire d'une canalisation ou destiné à être raccordé par une de ses extrémités à une tubulure dans laquelle circule un fluide,
- un raccord luer lock mâle comprenant une embase montée tournante sur le corps en coopérant par friction avec celui-ci, sans mouvement possible de translation, l'embase étant solidaire d'un corps creux tubulaire, dont la surface interne est munie d'un filetage et dont le centre est muni d'un cône luer mâle.

Ce connecteur se caractérise en ce qu'il est dépourvu de joint et en ce que la surface externe du corps et la surface interne de l'embase du raccord, coopérant par friction, ont une forme sphérique puis cylindrique.

Selon l'invention, le corps du connecteur est solidaire d'une canalisation ou destiné à être raccordé à une canalisation. Dans le premier cas de figure, on fait référence aux rampes, robinets ou raccord en Y dont l'extrémité présenterait directement la forme du corps du connecteur de l'invention. Dans le second cas de figure, on est dans l'hypothèse où le corps du connecteur de l'invention est raccordé par collage à une tubulure souple dans laquelle circule un fluide.

La caractéristique essentielle du connecteur est de présenter au niveau du corps et de l'embase du raccord, une forme sphérique puis cylindrique.

Que le connecteur soit utilisé pour la perfusion, ce qui représente la majorité des cas, ou le prélèvement, les zones sphérique puis cylindrique peuvent être agencées dans n'importe quel sens en direction de l'extrémité terminale du corps. On peut ainsi avoir la partie sphérique puis la partie cylindrique tout comme l'inverse. Quelle que soit la forme du corps, l'embase du raccord luer lock mâle présente une forme complémentaire coopérant par friction avec la forme correspondante du corps.

Les connecteurs dans lesquels le corps présente une forme sphérique puis cylindrique en direction de son extrémité terminale libre sont plus particulièrement décrits par la suite.

Lorsque le connecteur est destiné à être raccordé à une tubulure souple, la zone sphérique du corps est précédée d'une zone de liaison dont la surface externe présente une forme quelconque, généralement conique ou cylindrique. Dans ce cas, la tubulure souple est raccordée dans le canal formé au centre de la zone de liaison. En pratique, la zone de liaison est cylindrique et a un diamètre d₅, inférieur au diamètre d₁ de la portion de sphère du corps.

Lorsque le connecteur est solidaire d'une rampe, d'un robinet ou d'un Y, les parties sphérique puis cylindrique sont moulées directement au moment de leur fabrication.

Les différentes formes de réalisation du corps et du raccord correspondant vont maintenant être décrites plus en détail.

Dans un premier mode de réalisation,
- la surface externe du corps présente au moins deux tronçons successifs, respectivement :
   - un premier tronçon en forme de portion.de sphère de diamètre d₁,
   - un second tronçon cylindrique de diamètre d₂ inférieur à d₁
- la surface interne de l'embase présente :
   - une première zone en forme de portion de sphère de diamètre d₇ sensiblement égal à d₁,
   - une seconde zone tubulaire de diamètre d₃ légèrement inférieur à d₂.

Dans ce cas, l'étanchéité et donc le serrage du raccord sur le corps du connecteur est assurée par contact étroit entre le tronçon cylindrique du corps et le tronçon cylindrique correspondant de l'embase du raccord, dont le diamètre d₃ est légèrement inférieur à d₂ de manière à pouvoir être emboîtés l'un dans l'autre en force. La zone sphérique remplit la fonction d'assemblage et de clipage du corps sur l'embase.

Dans un mode de réalisation avantageux :
- la surface externe du corps présente au moins trois tronçons successifs, respectivement :
   - un premier tronçon cylindrique de diamètre d₄,
   - un second tronçon en forme de portion de sphère, le diamètre d₄ étant sensiblement égal au diamètre d₁ de la section la plus grande de la portion de sphère,
   - un troisième tronçon cylindrique de diamètre d₂ inférieur à d₁,
- la surface interne de l'embase présente :
   - une première zone en forme de portion de sphère de diamètre d₇ sensiblement égal à d₁,
   - une seconde zone tubulaire de diamètre d₃ légèrement inférieur à d₂.

Dans ce cas, le connecteur présente deux zones d'étanchéité. La première zone d'étanchéité est assurée par le contact étroit entre le troisième tronçon cylindrique et le tronçon cylindrique correspondant de l'embase du raccord, dont le diamètre d₃ est légèrement inférieur à d₂. La seconde zone d'étanchéité est quant à elle assurée par le contact étroit entre l'arête du premier tronçon cylindrique de diamètre d₄ du corps et la forme sphérique de l'embase de diamètre d₁. La zone sphérique garde sa fonction originelle d'assemblage et de clipage.

Quel que soit le mode de réalisation du connecteur, la zone sphérique de l'embase peut être précédée d'une zone avantageusement tubulaire de diamètre interne d₆ inférieur à d₁, cette zone étant destinée à venir enserrer soit la canalisation de la rampe soit la zone de liaison de diamètre d₅ du connecteur lorsque celui-ci est agencé à l'extrémité d'une tubulure souple. En pratique, d₅ est inférieur ou égal à d₆.

L'invention et les avantages qui en découlent ressortiront mieux des exemples de réalisation suivants, à l'appui des figures annexées.
La figure 1 est une vue en coupe du connecteur de l'invention.
La figure 2 est une vue en coupe du corps.
La figure 3 est une vue en coupe du raccord.

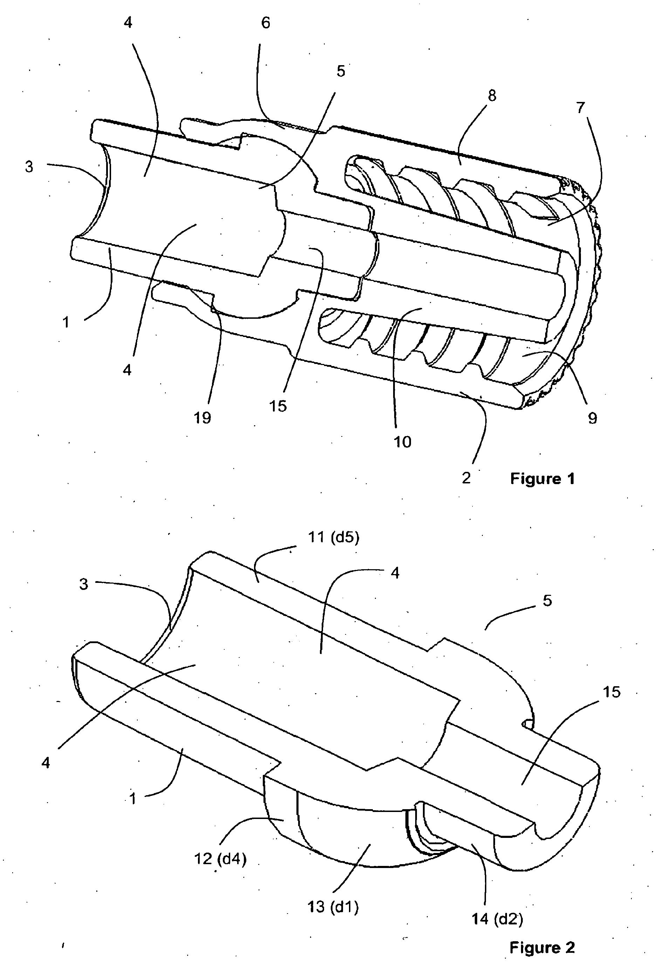

Sur la figure 1, on a représenté un connecteur luer mâle lock du type de celui de l'invention. Ce connecteur est constitué de deux pièces distinctes, respectivement un corps (1) et un raccord luer mâle (2).

Le corps (1) est destiné à être raccordé par son extrémité (3) à une tubulure de fluide non représentée. En pratique, la tubulure de fluide est raccordée au corps par emboîtement puis collage dans le canal (4). A son extrémité opposée (5), le corps coopère par friction avec l'embase (6) du raccord (2). En pratique, le contact exercé entre la surface interne de l'embase (6) et la surface externe de l'extrémité terminale (5) du corps (1) est à l'origine de la rotation du raccord par rapport au corps sans possibilité aucune de translation. Dans sa partie terminale, le raccord luer mâle est un raccord classique constitué d'un corps creux (7) comprenant une paroi (8), dont la surface interne (9) est munie d'un filetage permettant de visser et donc de bloquer le luer femelle après connexion. Le corps creux comprend en outre un canal central (10) conique luer mâle.

Sur la figure 2, on a représenté une coupe du corps (1). Selon l'invention, ce corps présente quatre zones distinctes successives, respectivement,
- en amont, une zone dite de liaison de surface externe tubulaire (11) de diamètre d₅,
- puis un premier tronçon cylindrique (12) de diamètre externe d₄ supérieur à d₅
- puis un second tronçon (13) en forme de portion de sphère de diamètre d₁ de la section la plus grande est sensiblement égal à d₄
- et un troisième tronçon (14) de forme tubulaire de diamètre d₂ inférieur à d₁.

Comme déjà dit, le corps est muni, en son centre, d'un canal (4) dans lequel circule le fluide. Ce canal présente une première section puis une section inférieure (15) de diamètre inférieur, sensiblement égale à la section du canal du luer mâle (10).

Le raccord luer lock va maintenant être décrit en détail.

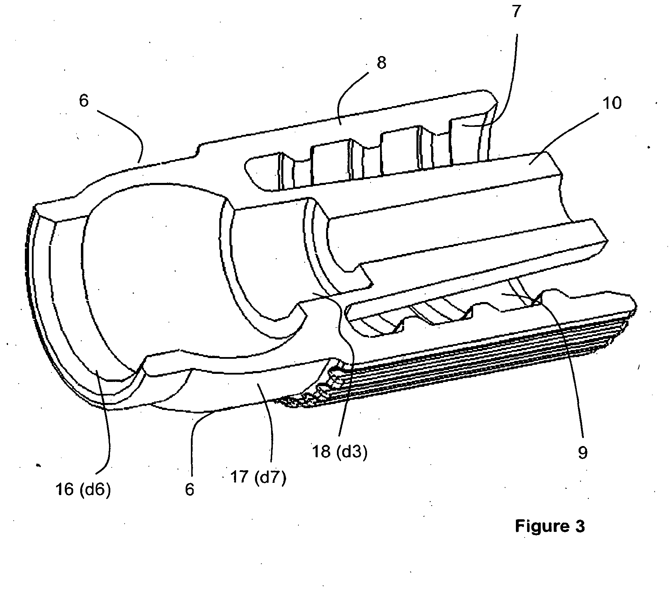

Comme représenté sur la figure 3, l'embase (6) présente une première zone (16) tubulaire, dont le diamètre de la surface interne d₆ est sensiblement égal au diamètre d₅ de la zone de liaison du corps. Par ailleurs, l'embase présente un premier tronçon (17) présentant une forme sphérique dont le diamètre d₇ est sensiblement égal à celui de la section la plus grande de la portion de sphère de diamètre d₁ du corps. Enfin, l'embase du raccord présente une seconde zone tubulaire (18) de forme tubulaire, dont le diamètre d₃ est sensiblement égal à celui du diamètre d₂ du corps (1).

De la sorte, le clipsage du raccord (2) sur le corps (1) créé deux zone d'étanchéité. La superposition de la zone cylindrique terminale (14) du corps (1) avec la zone correspondante (18) de l'embase assure l'étanchéité primaire du système en raison du diamètre d₃ <d₂. et donc du montage en force des deux éléments par emboitage. La zone d'interférence existant entre l'arête (19) de zone cylindrique (12) du corps et la zone sphérique (17) de l'embase assure une étanchéité secondaire.

L'invention et les avantages qui en découlent ressortent bien de la description qui précède. On note notamment une zone de friction présentant une forme spécifique permettant de garantir l'étanchéité du connecteur sans risque de déconnexion et en l'absence de joint.

## Revendications

1. Connecteur à usage médical comprenant :
- un corps (1) solidaire d'une canalisation ou destiné à être raccordé par une de ses extrémités (3) à une tubulure dans laquelle circule un fluide,
- un raccord (2) luer lock mâle comprenant une embase (6) montée tournante sur le corps (1) en coopérant par friction avec celui-ci, sans mouvement possible de translation, l'embase (6) étant solidaire d'un corps creux tubulaire (7), dont la surface interne (9) est munie d'un filetage et dont le centre est muni d'un cône luer mâle (10),
**caractérisé en ce qu'**il est dépourvu de joint et **en ce que** la surface externe du corps (1) et la surface interne de l'embase (6) du raccord (2), coopèrent par friction, la surface externe du corps (1) présentant en direction de son extrémité terminale libre deux tronçons successifs, respectivement :
• un premier tronçon (13) en forme de portion de sphère de diamètre d₁,
• un second tronçon (14) cylindrique de diamètre d₂ inférieur à d₁
- la surface interne de l'embase (6) présentant :
• une première zone en forme de portion de sphère (17) de diamètre d₇ sensiblement égal à d₁,
• une seconde zone (18) tubulaire de diamètre d₃ légèrement inférieur à d₂.

2. Connecteur selon la revendication 1, **caractérisé en ce que** :
- la surface externe du corps (1) présente au moins trois tronçons successifs, respectivement :
• un premier tronçon (12) cylindrique de diamètre d₄,
• un second tronçon (13) en forme de portion de sphère, le diamètre d₄ étant sensiblement égal au diamètre d₁ de la section la plus grande de la portion de sphère,
• un troisième tronçon cylindrique (14) de diamètre d2 inférieur à d₁,
- la surface interne de l'embase présente :
• une première zone en forme de portion de sphère (17) de diamètre d₇ sensiblement égal à d₁,
• une seconde zone tubulaire (18) de diamètre d₃ légèrement inférieur à d₂.

3. Connecteur selon l'une des revendications 1 ou 2, **caractérisé en ce que** la zone sphérique (17) de l'embase est précédée d'une zone tubulaire (16) de diamètre d₆ inférieure à d₁.

4. Connecteur selon l'une des revendications précédentes, **caractérisé en ce que** la zone sphérique (13) du corps (1) est précédée d'une zone de liaison(11) de diamètre d₅, dont la surface externe présente une forme quelconque.

5. Robinets équipés du connecteur objet des revendications 1 à 4.

6. Rampes équipées du connecteur objet des revendications 1 à 4.

7. Raccords en Y équipés du connecteur objet des revendications 1 à 4.

8. Lignes de perfusion équipées du connecteur objet des revendications 1 à 4.

9. Prolongateurs équipés du connecteur objet des revendications 1 à 4.

## Patentansprüche

1. Stecker zum medizinischen Gebrauch, Folgendes aufweisend:
- einen Körper (1), der mit einer Kanalleitung verbunden oder dazu bestimmt ist, mit einem seiner Enden (3) an eine Rohrleitung angeschlossen zu werden, in der ein Fluid zirkuliert,
- einen Luer-Steckeranschluss (2), der einen Ansatz (6) aufweist, der drehend am Körper (1), reibschlüssig mit diesem zusammenwirkend, ohne Translationsbewegungsmöglichkeit angebracht ist, wobei der Ansatz (6) mit einem rohrförmigen Hohlkörper (7) verbunden ist, dessen Innenfläche (9) mit einem Gewinde versehen ist, und dessen Zentrum mit einem Luer-Steckerkonus (10) versehen ist,
**dadurch gekennzeichnet, dass** er dichtungslos ist, und dass die Außenfläche des Körpers (1) und die Innenfläche des Ansatzes (6) des Anschlusses (2) reibschlüssig zusammenwirken, wobei die Außenfläche des Körpers (1) in der Richtung seines freien Abschlussendes zwei aufeinanderfolgende Teilabschnitte aufweist, und zwar:
• einen ersten Teilabschnitt (13) in Form eines Kugelabschnitts mit einem Durchmesser d₁,
• einen zweiten, zylindrischen Teilabschnitt (14) mit einem Durchmesser d₂, der kleiner ist als d₁,
- wobei die Innenfläche des Ansatzes (6) aufweist:
• einen ersten Bereich in Form eines Kugelabschnitts (17) mit einem Durchmesser d₇, der im Wesentlichen d₁ gleich ist,
• einen rohrförmigen zweiten Bereich (18) mit einem Durchmesser d₃, der etwas kleiner ist als d₂.

2. Stecker nach Anspruch 1, **dadurch gekennzeichnet, dass**:
- die Außenfläche des Körpers (1) mindestens drei aufeinanderfolgende Teilabschnitte aufweist, und zwar:
• einen ersten, zylindrischen Teilabschnitt (12) mit einem Durchmesser d₄,
• einen zweiten Teilabschnitt (13) in Form eines Kugelabschnitts, wobei der Durchmesser d₄ im Wesentlichen gleich dem Durchmesser d₁ des größten Abschnitts des Kugelabschnitts ist,
• einen dritten, zylindrischen Teilabschnitt (14) mit einem Durchmesser d₂, der kleiner ist als d₁,
- wobei die Innenfläche des Ansatzes aufweist:
• einen ersten Bereich in Form eines Kugelabschnitts (17) mit einem Durchmesser d₇, der im Wesentlichen gleich d₁ ist,
• einen zweiten, rohrförmigen Bereich (18) mit einem Durchmesser d₃, der etwas kleiner ist als d₂.

3. Stecker nach einem der Ansprüche 1 oder 2, **dadurch gekennzeichnet, dass** dem kugelförmigen Bereich (17) des Ansatzes ein rohrförmiger Bereich (16) mit einem Durchmesser d₆ vorgelagert ist, der kleiner als d₁ist.

4. Stecker nach einem der vorhergehenden Ansprüche, **dadurch gekennzeichnet, dass** dem kugelförmigen Bereich (13) des Körpers (1) ein Verbindungsbereich (11) mit einem Durchmesser d₅ vorgelagert ist, dessen Außenfläche eine beliebige Form aufweist.

5. Hähne, die mit dem Stecker nach einem der Ansprüche 1 bis 4 ausgestattet sind.

6. Verteiler, die mit dem Stecker nach einem der Ansprüche 1 bis 4 ausgestattet sind.

7. Y-Anschlüsse, die mit dem Stecker nach einem der Ansprüche 1 bis 4 ausgestattet sind.

8. Perfusionsleitungen, die mit dem Stecker nach einem der Ansprüche 1 bis 4 ausgestattet sind.

9. Verlängerungen, die mit dem Stecker nach einem der Ansprüche 1 bis 4 ausgestattet sind.

## Claims

1. Connector for medical use comprising:
- a body (1) joined to a line or adapted to be connected at one end (3) to a tubing in which a fluid flows,
- a male luer lock connection (2) comprising a base (6) rotary mounted on the body (1) and cooperating by friction with said body (1), without any possible translational movement, the base (6) being joined to a hollow tubular body (7), having a threaded inner surface (9) and a central male luer cone (10),
wherein the connector has no seal and wherein the outer surface of the body (1) and an inner surface of the base (6) of the connection (2) cooperate by friction, the outer surface of the body (1) having towards its free terminal end two successive segments, respectively:
• a first segment (13) in the form of a portion of sphere having a diameter d₁, and
• a second cylindrical segment (14) having a diameter d₂ smaller than d₁; and
- the inner surface of the base (6) having:
• a first zone in the form of a portion of sphere (17) having a diameter d₇ roughly equal to d₁, and
• a second tubular zone (18), having a diameter d₃ slightly smaller than d₂.

2. Connector according to claim 1, wherein:
- the outer surface of the body (1) further includes at least three successive segments :
• a first cylindrical segment (12) having a diameter d₄,
• a second segment (13) in the form of a portion of sphere, the diameter d₄ being roughly equal to the diameter d₁ of the longest section in the form of a portion of sphere.
• A third cylindrical segment (14) having a diameter d₂ smaller than d₁,
- the inner surface of the base (6) having:
• a first zone in the form of a portion of sphere (17) having a diameter d₇ roughly equal to d₁, and
• a second tubular zone (18), having a diameter d₃ slightly smaller than d₂.

3. Connector according to one of preceding claims 1 or 2, wherein the spherical zone (17) of the base (6) is preceded by a tubular zone (16) having diameter d₆ smaller than d₁.

4. Connector according to one of the preceding claims, wherein the spherical zone (13) of the body (1) is preceded by a connecting zone (11) having a diameter d₅ of which the outer surface has a random shape.

5. A valve equipped with the connector according to one of the claims 1 to 4.

6. A ramp equipped with the connector according to one of the claims 1 to 4.

7. A Y-piece connector equipped with the connector according to one of the claims 1 to 4.

8. A perfusion tube equipped with the connector according to one of the claims 1 to 4.

9. An extension equipped with the connector according to one of the claims 1 to 4.
